# EUROPEAN PATENT SPECIFICATION

(11) **EP 3 398 570 B1**
(45) Date of publication and mention of the grant of the patent: **13.05.2020**
(21) Application number: 18165395.7
(22) Date of filing: 03.04.2018
(51) Int. Cl.: A61F 5/01

(54) **BRACE AND INSERTION-FITTING MEMBER USED THEREFORE**
STÜTZE UND DAFÜR VERWENDETES EINSTECKVERBINDUNGSELEMENT
ATTELLE ET ÉLÉMENT DE SUPPORT D'INSERTION UTILISÉ ASSOCIÉ

(30) Priority: 11.04.2017 JP 2017078544
(43) Date of publication of application: 07.11.2018
(73) Proprietor: Toyota Jidosha Kabushiki Kaisha, Aichi 471-8571 (JP)
(72) Inventor: TAKEDA, Takahiro, Toyota-shi, Aichi 471-8571 (JP); ASAYAMA, Masakazu, Toyota-shi, Aichi 471-8571 (JP)
(74) Representative: TBK

(56) References cited:
- US-A- 5 372 574
- US-A1- 2004 015 112
- US-A1- 2011 009 788

## Description

### BACKGROUND

The present disclosure relates to a brace and an insertion-fitting member used therefore.

An assistance brace that is attached to a leg of a user and assists walking of the user has been known. For example, in an assistance brace disclosed in Japanese Patent Application Publication No. 2007-54086, a lower-thigh frame attached to a lower thigh of a user is rotatably connected to a sole frame on which the user places his/her sole. In this assistance brace, assisting motions are controlled by driving a link mechanism by using a motor so that a plantar-flexion/dorsiflexion motion of an ankle joint is assisted and the plantar-flexion/dorsiflexion motion of the ankle joint does not exceed a movable range.

### SUMMARY

The present inventors have found the following problem. A power-driven assistance brace tends to have a large size and hence its usability as a brace used for a joint of a human body including an ankle joint is not satisfactory. Even in the case of a passive assistance brace that does not provide power-driven assistance to joint motions, depending on a disease state of a user, it is necessary to regulate a movable range of the assistance brace so that the user does not excessively bend his/her joint. However, when a regulation range within which a user is allowed to bend his/her joint is made narrower than a range within which the user can physically and naturally bend the joint, a large impact is given to a regulation member at both ends of the regulated range by a bending force of the joint. When the regulation member is damaged or worn out by such impacts, it becomes impossible to appropriately manage the allowable motion range and, in some cases, such damage interferes with a rehabilitation plan.

The present disclosure has been made to solve the above-described problem and an object thereof is to provide a brace capable of accurately and easily maintaining a movable range of a joint within a desired regulation range and an insertion-fitting member used therefore.

The present invention relates to a brace as set out in claim 1.

By regulating the rotation range of the regulation part by using the insertion-fitting member which is insertion-fitted in a replaceable manner as described above, it is possible to replace the insertion-fitting member with a new one when the projecting part thereof is worn out or damaged. Therefore, it is possible to accurately regulate the movable range of the joint to a target range. Consequently, it is possible to carry out a rehabilitation plan without a hitch. Further, since the regulation part and the insertion-fitting member make surface contact, it is possible to reduce the wear of the insertion-fitting member and prevent the damage thereof.

In the above-described configuration, the insertion-fitting member, which is insertion-fitted into the insertion-fitting receiving part, is selected from a set of a plurality of insertion-fitting members according to a desired regulated rotation range, the plurality of insertion-fitting members including projecting parts having projecting lengths different from each other in the circumferential direction. By preparing insertion-fitting members having different projecting lengths, it is possible to easily change the desired regulated rotation range according to a wearer's situation.

Further, the insertion-fitting member may be able to be insertion-fitted into the insertion-fitting receiving part in a state in which the first and second members are already attached to the wearer. By configuring the brace in the above-described manner, it is possible, when the insertion-fitting member is worn out or damaged, to replace the insertion-fitting member with a new one without detaching the joint regulation apparatus from the wearer, even when the wearer is doing rehabilitation training.

Further, the insertion-fitting member can be configured so as to include: a first insertion-fitting member including a first projecting part configured to come into contact with the regulation part at one end of a rotating motion thereof; and a second insertion-fitting member including a second projecting part configured to come into contact with the regulation part at the other end of the rotating motion thereof. By adopting the configuration in which the regulation part is regulated at one end and the other end thereof by using different insertion-fitting members as described above, it is possible to replace these insertion-fitting members separately from each other according to their worn-out states or the like. Further, by using insertion-fitting members having different projecting lengths, it is possible, when the joint regulation apparatus is applied to, for example, an ankle joint, to adjust a movable range for plantar-flexion and a movable range for dorsiflexion separately from each other.

Further, the joint mechanism may include a lever configured to press down the insertion-fitting member in a direction toward the insertion-fitting receiving part after the insertion-fitting member is inserted into the insertion-fitting receiving part. By adopting the lever, it is possible to firmly fix the insertion-fitting member in the insertion-fitting receiving part and also possible to easily and swiftly replace the insertion-fitting member. Further, by providing a positioning mechanism for stopping the lever in a predetermined position in the joint mechanism, it is possible to stabilize the lever position and contribute to an improvement in feeling of operation.

Further, one of the first and second members is preferably a lower-thigh frame attached to a lower thigh of the wearer, and the other of the first and second members is preferably a sole plate on which the wearer places his/her sole. Further, the joint mechanism is preferably used in such a manner that the rotation range is determined so that a range of a plantar-flexion motion and a dorsiflexion motion of an ankle joint of the wearer is regulated. In the regulation for motions of an ankle joint, large impacts tend to occur particularly at both ends of the regulation range and hence a number of advantageous effects can be enjoyed by the above-described function which enables the insertion-fitting member to be replaced.

Another exemplary aspect is an insertion-fitting member configured to insertion-fitted into an insertion-fitting receiving part of the above-described joint regulation apparatus in a replaceable manner. The insertion-fitting member, which is processed (e.g., machined) so that it makes surface contact with the regulation part of the joint regulation apparatus, has high durability and hence can be used for a long time.

According to the present disclosure, it is possible to provide a brace capable of accurately and easily maintaining a movable range of a joint in a desired regulation range and an insertion-fitting member used therefore.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a perspective view of an external appearance of an ankle joint regulation apparatus according to an embodiment;
Fig. 2 is a perspective view of an external appearance of a joint mechanism;
Fig. 3 is a cross section showing a configuration of a main part of the joint mechanism;
Fig. 4 is a perspective view of an external appearance of stopper blocks showing a state in which they are insertion-fitted into a holder;
Fig. 5 is a front view of a joint mechanism for showing a lever operation;
Fig. 6 is a cross section taken along a line A-A in Fig. 5;
Fig. 7 is a perspective view of an external appearance of a stopper block;
Fig. 8 is a schematic diagram schematically showing a reference state of a joint mechanism;
Fig. 9 is a schematic diagram showing a state of a plantar-flexion rotation of the joint mechanism; and
Fig. 10 is a schematic diagram showing a state of a dorsiflexion rotation of the joint mechanism.

### DESCRIPTION OF EMBODIMENTS

The present disclosure is explained hereinafter by using embodiments. However, the present disclosure according to the claims is not limited to the below-shown embodiments. Further, each of the components explained in the embodiments is not necessarily indispensable as means for solving the problem.

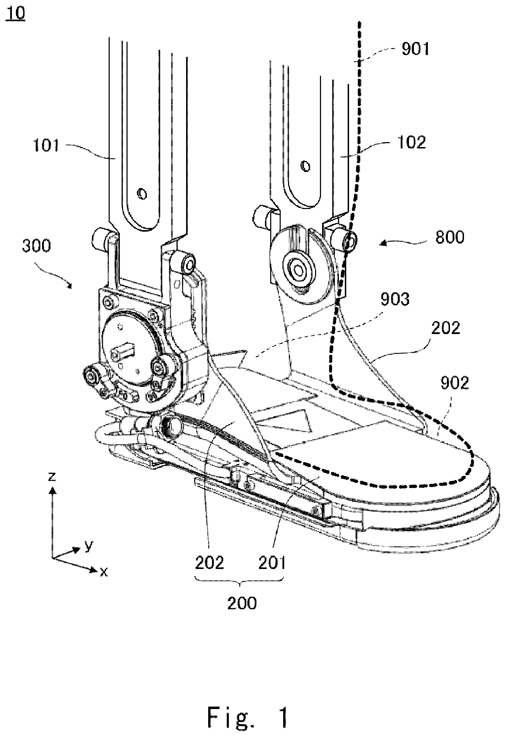

Fig. 1 is a perspective view of an external appearance of an ankle joint regulation apparatus 10 according to an embodiment. The ankle joint regulation apparatus 10 is a regulation apparatus that regulates a movable range of an ankle joint 903 that connects a bone of a lower-thigh (i.e., a shinbone and a fibula) of a wearer with his/her anklebone. The ankle joint regulation apparatus 10 is mainly composed of a lower-thigh frame 101 that is located on a side of a lower-thigh bone and supports a lower thigh 901, a sole frame 200 that is located on a side of the anklebone and supports a part from an ankle to a tip of a foot 902, and a joint mechanism 300 that supports these two frames so that they can swing within a swing range regulated along a movable direction of the ankle joint 903.

The sole frame 200 includes a bottom plate 201 having a placement surface on which the wearer places his/her sole, and side plates 202 vertically disposed on sides of the bottom plate 201. More specifically, the joint mechanism 300 connects a lower end of the lower-thigh frame 101 with the outer-side side plate 202 in such a manner that they can swing. The sole frame 200 is attached to the foot 902 thorough an attachment belt or the like (not shown). For example, when the wearer wears a shoe-like cup sole, the sole frame 200 may be constructed so as to fix the cup sole. As described later, the joint mechanism 300 has such a structure that the joint mechanism 300 swings within a range that is narrower than a range within which the ankle joint 903 can be physically and naturally bent.

The ankle joint regulating apparatus 10 further includes a lower-thigh frame 102 located on a side of the lower thigh 901 opposite to the side on which the lower-thigh frame 101 is located. That is, the lower-thigh frames 101 and 102 are attached so as to sandwich the lower thigh 901 therebetween and support the lower thigh 901. Note that the lower-thigh frames 101 and 102 are attached to the lower thigh 901 thorough an attachment belt or the like (not shown).

The ankle joint regulating apparatus 10 includes a connecting mechanism 800 that connects the lower-thigh frame 102 with the inner-side side plate 202 in such a manner that they can swing. Unlike the joint mechanism 300, the connecting mechanism 800 does not include a structure for regulating the swing. That is, the movable range of the ankle joint 903 is not regulated by the connecting mechanism 800 located on the inner side thereof, but is instead regulated by the joint mechanism 300 located on the outer side thereof. By adopting the above-described structure, the regulation range can be conveniently adjusted without detaching the ankle joint regulating apparatus 10 from the wearer.

Note that in Fig. 1, it is assumed that the diseased leg is the right leg and a state in which the joint mechanism 300 is disposed on the outer side of the right leg is shown. In this embodiment, the ankle joint regulating apparatus 10 is a regulation apparatus for a right leg. However, needless to say, it is possible to manufacture a regulation apparatus for a left leg in which the joint mechanism 300 is disposed on the outer side of the left leg. Further, as shown in the figures, a direction from an ankle toward a toe is defined as a positive direction on an x-axis, and a direction toward the inner side on a plane (i.e., a surface) of the bottom plate 201 is defined as a positive direction on a y-axis. Further, a direction toward an upper body along the thigh is defined as a positive direction on a z-axis. The same coordinate system as that shown in Fig. 1 is shown in each of the subsequent drawings to clarify each direction.

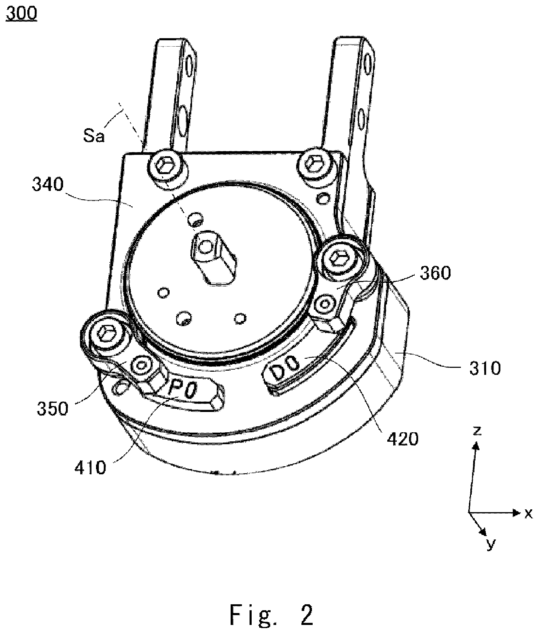

Fig. 2 is a perspective view of an external appearance of the joint mechanism 300. In particular, Fig. 2 shows a state in which a cover 340 is attached to the joint mechanism 300. In actual use, the cover 340 is attached to the joint mechanism 300 in order to prevent dust and the like from entering into the mechanism as shown in the figure. The joint mechanism 300 includes a holder 310 and stopper blocks 410 and 420 in addition to the cover 340. Note that the stopper block 410 and the holder 310 are examples of the insertion-fitting member and the insertion-fitting receiving part, respectively.

The holder 310 is fixed to the lower-thigh frame 101. The holder 310 is formed by, for example, carving it out of an aluminum block. Note that the holder 310 may be connected to the lower-thigh frame 101 so that the holder 310 is allowed to slightly rotate around the x-axis with respect to the lower-thigh frame 101. A lever 350 has a function of pressing down the stopper block 410, which has been attached in the holder 310 by insertion, in a direction toward the holder to prevent the stopper block 410 from being removed. Similarly, a lever 360 has a function of pressing down the stopper block 420, which has been attached in the holder 310 by insertion, in a direction toward the holder to prevent the stopper block 420 from being removed. Specific structures of the stopper blocks 410 and 420 and the levers 350 and 360 are described later.

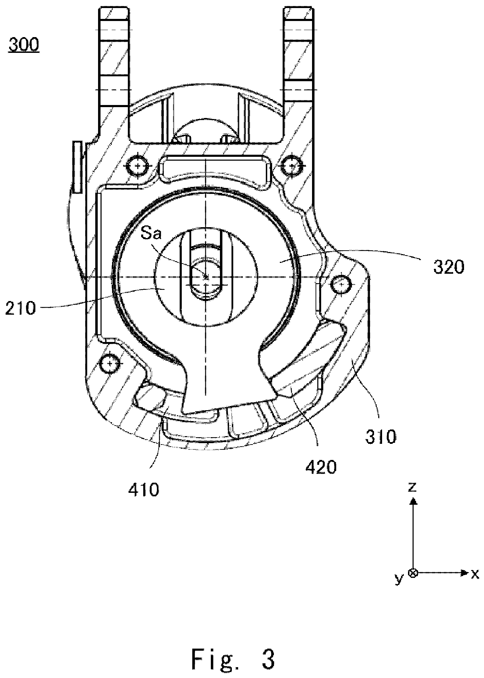

Fig. 3 is a cross section showing a configuration of a main part of the joint mechanism 300 when the holder 310 is cut on a plane parallel to the xz-plane. That is, Fig. 3 shows a state of an internal space of the holder 310. A swing shaft 320 is housed in the internal space of the holder 310. Note that the swing shaft 320 is an example of the regulation part.

The swing shaft 320 is fixed to the side plates 202 of the sole frame 200 through a coupling member 210. The swing shaft 320 is pivotally supported on the holder 310 in such a manner that it can swing around a swing axis Sa. That is, when the wearer performs a dorsiflexion motion and a plantar-flexion motion by moving his/her ankle joint, the sole frame 200 follows the motions and hence the swing shaft 320 swings around the swing axis Sa with respect to the holder 310.

The stopper block 410 is insertion-fitted into the holder 310 from the front thereof and regulates, regarding the swing range of the swing shaft 320, the swing angle of the plantar-flexion motion. The stopper block 420 is insertion-fitted into the holder 310 from the front thereof and regulates, regarding the swing range of the swing shaft 320, the swing angle of the dorsiflexion motion. Specific structures and specific motions are described later.

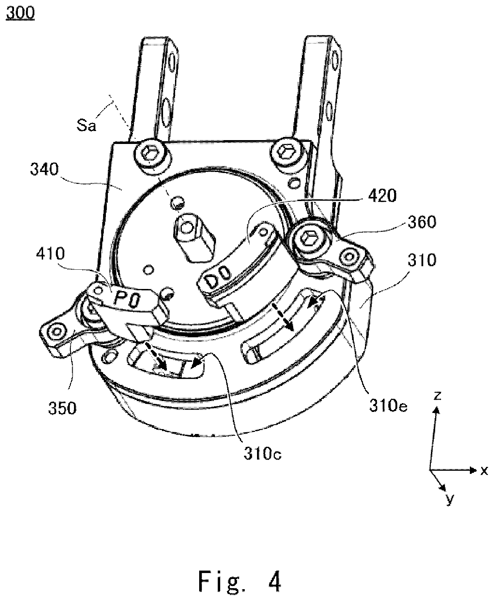

Fig. 4 is a perspective view of an external appearance of the stopper blocks 410 and 420 showing a state in which they are insertion-fitted into the holder 310. Each of the stopper block 410 for plantar flexion and the stopper block 420 for dorsiflexion is replaceable (i.e., removable) for the holder 310.

An insertion-fitting hole 310c, in which the stopper block 410 for plantar-flexion is inserted, and an insertion-fitting hole 310e, in which the stopper block 420 for dorsiflexion is inserted, are formed on the front side of the holder 310 and in the cover 340 covering the front of the holder 310. The insertion-fitting holes 310c and 310e are formed by hollowing out them so that their shapes conform to the circumferences of the stopper blocks 410 and 420, respectively, and have roughly elliptical shapes that extend along a circumference centered on the swing axis Sa.

The stopper block 410 is inserted into the insertion-fitting hole 310c in a direction indicated by a dotted-line arrow. Note that the lever 350 is rotated to a position in which the lever 350 retreated from the insertion-fitting hole 310c before the insertion of the stopper block 410. Similarly, the stopper block 420 is inserted into the insertion-fitting hole 310e in a direction indicated by a dotted-line arrow. The lever 360 is rotated to a position in which the lever 360 is retreated from the insertion-fitting hole 310e before the insertion of the stopper block 420.

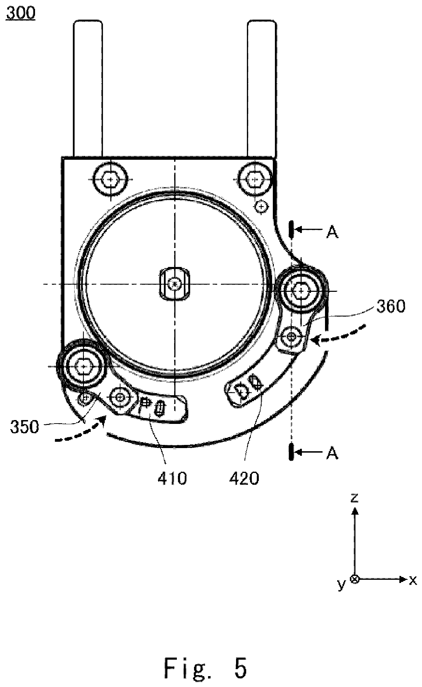

Fig. 5 is a front view of the joint mechanism 300 for showing a lever operation. After the stopper block 410 is inserted into the insertion-fitting hole 310c, the lever 350 is rotated in a direction indicated by a dotted-line arrow, so that the lever 350 presses down the stopper block 410 in the positive direction on the y-axis. Similarly, after the stopper block 420 is inserted into the insertion-fitting hole 310e, the lever 360 is rotated in a direction indicated by a dotted-line arrow, so that the lever 360 presses down the stopper block 420 in the positive direction on the y-axis.

The stopper blocks 410 and 420 can be removed by performing the operations explained above with reference to Figs. 4 and 5 in a reversed order. Needless to say, only one of the stopper blocks may be attached or removed. By adopting the above-described levers 350 and 360, it is possible to firmly fix the stopper blocks 410 and 420 in the holder 310 and also possible to easily and swiftly replace them.

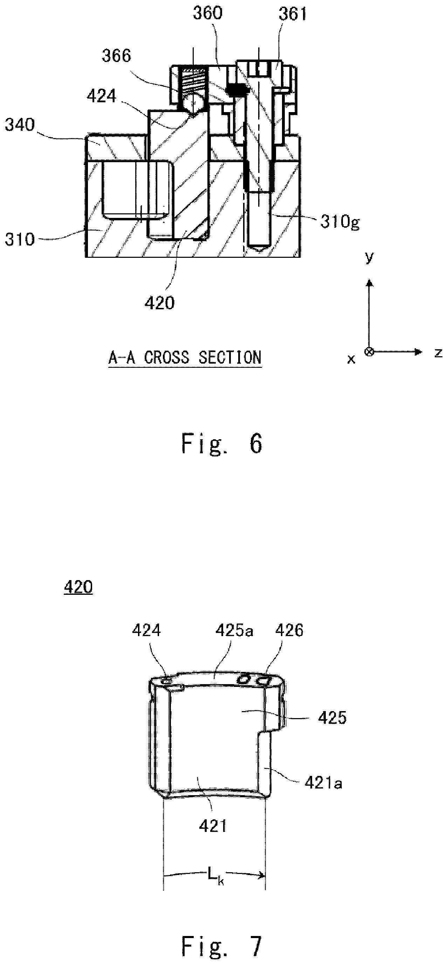

Fig. 6 is a cross section taken along a line A-A in Fig. 5, and shows a relation between the stopper block 420 attached in the holder 310 and the lever 360. Note that a relation between the stopper block 410 and the lever 350 is similar to the relation between the stopper block 420 and the lever 360, and therefore its explanation is omitted.

The lever 360 is supported on the holder 310 in such a manner that the lever 360 can rotate around a lever shaft 361 that is screwed into a screw hole 310g formed in the holder 310. A plunger ball 366 is partially buried in the tip of the lever 360 and is urged (i.e., pressed) in the negative direction on the y-axis.

When the lever 360 is rotated and its tip is positioned above the stopper block 420, which has been insertion-fitted in the holder 310, the plunger ball 366 is engaged in a cone-shaped positioning hole 424 formed in the stopper block 420. Once the plunger ball 366 is engaged in the positioning hole 424, the lever 360 remains stopped and stabilized unless it is rotated by a relatively strong force. The above-described positioning mechanism using the positioning hole 424 and the plunger ball 366 stabilizes the position of the lever 360 and contributes to an improvement in feeling that a user has when he/she operates the lever.

Fig. 7 is a perspective view of an external appearance of the stopper block 420. A structure of the stopper block 410 is similar to that of the stopper block 420 and therefore its explanation is omitted.

The stopper block 420 has a shape which seems as if it was formed by cutting out a part of a cylindrical wall. The stopper block 420 is mainly composed of a projecting block part 421 and a knob part 425. The projecting block part 421 functions as a projecting part of the stopper block 420. The projecting block part 421 is a part of the stopper block 420 that is, when the stopper block 420 is insertion-fitted into the holder 310, disposed in the internal space of the holder 310 and collides with the swing shaft 320. Further, the knob part 425 is a part that is disposed on the side of the cover 340. The stopper block 420 is formed by, for example, carving it out of an aluminum block. The projecting block part 421 and the knob part 425 may be carved out as one integral component, or may be separately produced and integrated into one component by an adhesive or the like.

The knob part 425 is a part that is engaged in the above-described insertion-fitting hole 310e and also serves as a part that a user grasps when he/she replaces the stopper block 420. An inscribed mark 426, which is a mark indicating a property of the stopper block 420, is formed on a top surface 425a, i.e., an upper surface of the knob part 425. The inscribed mark 426 is provided so that a user can recognize, at a glance, whether the stopper block is for plantar-flexion or for dorsiflexion, and/or how long an effective length Lₖ is (or how large a regulated swing angle is). The effective length Lₖ and the swing angle are explained below. Further, the above-described positioning hole 424 is formed on the top surface 425a.

When the central axis of the positioning hole 424 is defined as a reference line, the projecting block part 421 extends, when the stopper block 420 is insertion-fitted in the holder 310, along a circumference centered on the swing axis Sa of the swing shaft 320. Further, the length of the projecting block part 421 is accurately adjusted to a predetermined effective length Lₖ. That is, the effective length L_{K} corresponds to the projecting length of the projecting part. Further, the end face of the tip of the projecting block part functions as an impact receiving surface 421a that comes into contact with the swing shaft 320.

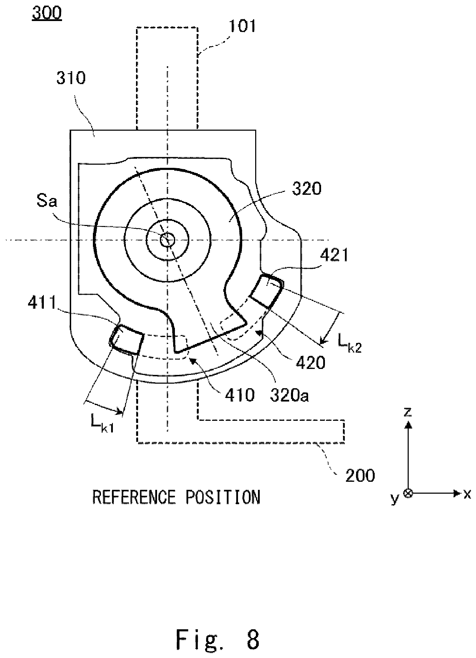

Fig. 8 is a schematic diagram schematically showing a reference state of the joint mechanism 300. Note that the reference state means a state in which the wearer stands upright. In this state, the sole frame 200 is perpendicular to the lower-thigh frame 101.

When the stopper blocks 410 and 420 are insertion-fitted into the insertion-fitting holes 310c and 310e, respectively, as shown in Figs. 2 and 4, the swing shaft 320 and the projecting block parts 411 and 421 have a positional relation therebetween as shown in Fig. 8 in the internal space of the holder 310 in the reference state.

The swing shaft 320 includes an arm part 320a extending in a radial direction with respect to the swing axis Sa. When the swing shaft 320 swings, the tip of the arm part 320a swings on the circumference on which the projecting block parts 411 and 421 are arranged within a range between the projecting block parts 411 and 421.

The projecting block part 411 extends an effective length Lₖ₁ in the circumferential direction along which the tip of the arm part 320a swings. Similarly, the projecting block part 421 extends an effective length Lₖ₂ in the circumferential direction along which the tip of the arm part 320a swings. That is, the swing range for plantar-flexion motions is regulated according to the effective length Lₖ₁, i.e., the projecting length of the projecting block part 411 and the swing range for dorsiflexion motions is regulated according to the effective length Lₖ₂, i.e., the projecting length of the projecting block part 421.

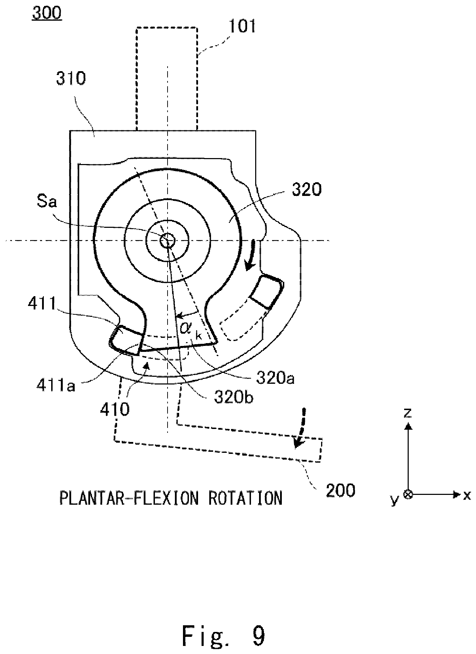

Fig. 9 is a schematic diagram showing a state of a plantar-flexion rotation of the joint mechanism 300. An impact surface 320b, which collides with an impact receiving surface 411a, i.e., an end face of the projecting block part 411, is formed on one side of the arm part 320a. When a wearer performs a plantar-flexion motion, the sole frame 200 swings in a direction indicated by a dotted-line arrow in the figure. As a result, the swing shaft 320 swings in a direction indicated by a bold-line arrow. Then, when the swing shaft 320 swings around the swing axis Sa by αₖ degrees, the impact surface 320b collides with the impact receiving surface 411a. That is, the joint mechanism 300 regulates the swing range so that the swing shaft 320 can swing on the plantar-flexion side by αₖ degrees or smaller.

The stopper block 410 for plantar-flexion is selected by a user from a set of a plurality of stopper blocks with projecting block parts 411 having different effective lengths Lₖ₁ according to the desired regulated swing range on the plantar-flexion side. A surface treatment has been performed on each of the impact surface 320b of the swing shaft 320 and the impact receiving surface 411a of the stopper block 410 so that the impact surface 320b collides with the impact receiving surface 411a in surface contact regardless of which of the stopper blocks having different effective lengths Lₖ₁ is inserted into the insertion-fitting hole 310c.

For example, when the impact receiving surface 411a is processed (e.g., machined) into a flat surface along the radial direction centered on the swing axis Sa, the impact surface 320b is also processed (e.g., machined) into a flat surface along the radial direction centered on the swing axis Sa. Since the impact surface 320b collides with the impact receiving surface 411a in surface contact irrespective of the desired regulated swing angle αₖ, it is possible to disperse an impact force that is caused at the time of a collision and thereby to reduce damage and wear of the stopper block 410. Note that the set of stopper blocks may include a stopper block having an effective length Lₖ₁ by which the swing angle αₖ on the plantar-flexion side is regulated to 0 degrees.

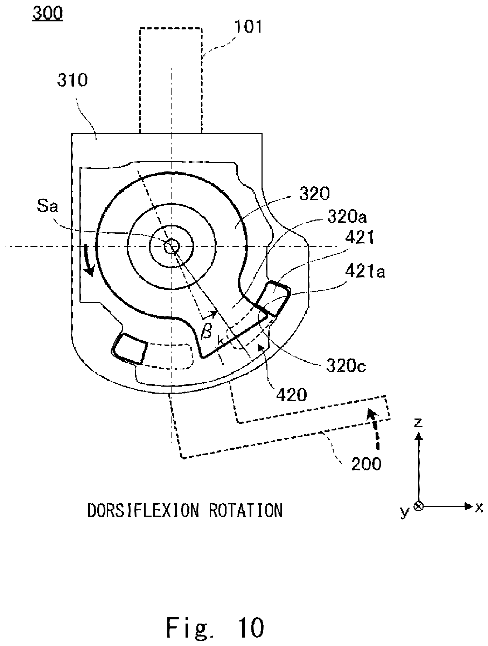

Fig. 10 is a schematic diagram showing a state of a dorsiflexion rotation of the joint mechanism 300. An impact surface 320c, which collides with an impact receiving surface 421a, i.e., an end face of the projecting block part 421, is formed on the other side of the arm part 320a, i.e., a side of the arm part 320a opposite to the side on which the impact surface 320b is formed. When a wearer performs a dorsiflexion motion, the sole frame 200 swings in a direction indicated by a dotted-line arrow in the figure. As a result, the swing shaft 320 swings in a direction indicated by a bold-line arrow. Then, when the swing shaft 320 swings around the swing axis Sa by βₖ degrees, the impact surface 320c collides with the impact receiving surface 421a. That is, the joint mechanism 300 regulates the swing range so that the swing shaft 320 can swing on the dorsiflexion side by βₖ degrees or smaller.

Similarly to the stopper block 410 for plantar-flexion, the stopper block 420 for dorsiflexion is selected by a user from a set of a plurality of stopper blocks with projecting block parts 421 having different effective lengths Lₖ₂ according to the desired regulated swing range on the dorsiflexion side. A surface treatment has been performed on each of the impact surface 320c of the swing shaft 320 and the impact receiving surface 421a of the stopper block 420 so that the impact surface 320c collides with the impact receiving surface 421a in surface contact regardless of which of the stopper blocks having different effective lengths Lₖ₂ is inserted into the insertion-fitting hole 310e.

For example, when the impact receiving surface 421a is processed (e.g., machined) into a flat surface along the radial direction centered on the swing axis Sa, the impact surface 320c is also processed (e.g., machined) into a flat surface along the radial direction centered on the swing axis Sa. Since the impact surface 320c collides with the impact receiving surface 421a in surface contact irrespective of the desired regulated swing angle βₖ, it is possible to disperse an impact force that is caused at the time of a collision and thereby to reduce damage and wear of the stopper block 420. Note that the set of stopper blocks may include a stopper block having an effective length Lₖ₂ by which the swing angle βₖ on the dorsiflexion side is regulated to 0 degrees. By selecting the stopper block 410 by which the swing angle αₖ on the plantar-flexion side is regulated to 0 degrees and the stopper block 420 by which the swing angle βₖ on the dorsiflexion side is regulated to 0 degrees, it is possible to fix the joint angle at a reference position.

As has been explained so far, the joint mechanism 300 can regulate each of the swing angle αₖ on the plantar-flexion side and the swing angle βₖ on the dorsiflexion side to a desired swing range independently of each other by selecting stopper blocks 410 and 420 having appropriate effective lengths Lₖ₁ and Lₖ₂, respectively, and fixing the selected stopper blocks to the holder 310. In the ankle joint regulation apparatus 10 according to this embodiment, since the swing range is regulated to a range narrower than a range in which a wearer can physically and naturally bend his/her foot as an ankle joint function, the impact surfaces 320b and 320c collide with the impact receiving surfaces 411a and 421a, respectively, with large forces. However, the ankle joint regulation apparatus 10 is configured so that the stopper blocks 410 and 420 are replaceable. Therefore, it is possible to accurately maintain the desired regulated swing range by replacing them with new ones according to their worn-out situation or the like. Further, the ankle joint regulation apparatus 10 is configured so that a wearer can easily insert or remove the stopper blocks 410 and 420 without detaching the ankle joint regulation apparatus 10 from the wearer. Therefore, the wearer can proceed with rehabilitation training without a hitch.

In the above-explained embodiments, an ankle joint regulation apparatus attached to an ankle joint, in which relatively large impacts occur in both ends of the regulated swing range, is explained. However, use of the above-described joint mechanism is not limited to use for ankle joint regulation apparatuses. That is, the above-described joint mechanism can be used for joint regulation apparatuses used for any kinds of joints, provided that the joint mechanism supports a first member attached to a part of a body located on a side of one of bones and a second member attached to a part of the body located on a side of the other bone so that they can swing in a regulated swing range. Further, the relative rotation of the first and second members may be assisted by an actuator such as a motor.

An aspect of the above-described ankle joint regulation apparatus is summarized. A joint regulation apparatus configured to regulate a movable range of a joint connecting a first bone part of a wearer with a second bone part of the wearer, including: a first member configured to be attached to a part of a body located on the first bone side; a second member configured to be attached to a part of the body located on the second bone side; and a joint mechanism configured to support the first and second members so that the first and second members can swing with respect to each other along a movable direction of the joint, in which the joint mechanism includes: a holder integrally provided with the first member; a regulation member including a projection part formed on a tip side, the regulation member being configured to be insertion-fitted into the holder in a replaceable manner; and a swing member configured to swing in an integrated manner with the second member, the swing member being further configured to come into contact with the projecting part of the regulation member at at least one of one end and the other end of a swinging motion, so that its swing range is regulated, and the projecting part is disposed so as to extend, when the regulation member is insertion-fitted into the holder, in a circumferential direction with respect to a swing axis of the swing member and is configured to make surface contact with a contact part of the swing member in the circumferential direction.

From the present disclosure thus described, it will be obvious that the embodiments of the present disclosure may be varied in many ways. Such variations are not to be regarded as a departure from the spirit and scope of the present disclosure, and all such modifications as would be obvious to one skilled in the art are intended for inclusion within the scope of the following claims.

## Claims

1. A brace (10) comprising:
a first member (101) configured to be attached to a part of a body located along a first bone part of a wearer;
a second member (200) configured to be attached to a part of a body located along a second bone part of the wearer; and
a joint mechanism (300) configured to support the first and second members (101, 200) so that the first and second members (101, 200) can rotate with respect to each other around a rotation axis, wherein
the brace (10) further comprises an insertion-fitting member (410,420) configured to be insertion-fitted into the joint mechanism (300) in such a manner that the insertion-fitting member (410,420) can be fixed to and removed from the joint mechanism (300),
the joint mechanism (300) comprises:
an insertion-fitting receiving part (310c,310e) integrally provided with the first member (101), the insertion-fitting receiving part (310c,310e) being configured to enable the insertion-fitting member (410,420) to be insertion-fitted into the joint mechanism (300); and
a regulation part (320) integrally provided with the second member (200), the regulation part (320) being disposed around the rotation axis in the second member (200), and
the regulation part (320), which rotates around the rotation axis in an integrated manner with a rotation of the second member (200), makes surface contact with a projecting part (411,421) of the insertion-fitting member (410,420) fixed to the insertion-fitting receiving part (310c,310e) on a trajectory of the rotation of the regulation part (320), so that a range of the rotation of the second member (200) is regulated, the projecting part (411,421) extending in a circumferential direction with respect to the rotation axis, **characterized in that**
the insertion-fitting member (410,420), which is insertion-fitted into the insertion-fitting receiving part (310c,310e), is selected from a set of a plurality of insertion-fitting members (410,420) according to a desired regulated rotation range, the plurality of insertion-fitting members (410,420) comprising projecting parts (411,421) having projecting lengths different from each other, and
the joint mechanism comprises a lever (350,360) configured to press down the insertion-fitting member (410,420) in a direction toward the insertion-fitting receiving part (310c,310e) after the insertion-fitting member (410,420) is inserted into the insertion-fitting receiving part (310c,310e).

2. The brace (10) according to Claim 1, wherein the insertion-fitting member (410,420) is able to be insertion-fitted into the insertion-fitting receiving part (310c,310e) in a state in which the first and second members (101, 200) are already attached to the wearer.

3. The brace (10) according to Claims 1 or 2, wherein the insertion-fitting member (410,420) comprises:
a first insertion-fitting member (410) comprising a first projecting part (411) configured to come into contact with the regulation part (320) at one end of a rotating motion thereof; and
a second insertion-fitting member (420) comprising a second projecting part (421) configured to come into contact with the regulation part (320) at the other end of the rotating motion thereof.

4. The brace (10) according to Claim 1, wherein the joint mechanism (360,424) comprises a positioning mechanism (366) for stopping the lever (360) in a predetermined position.

5. The brace (10) according to any one of Claims 1 to 4, wherein
one of the first and second members (101, 200) is a lower-thigh frame (101) attached to a lower thigh of the wearer,
the other of the first and second members (101, 200) is a sole plate (200) on which the wearer places his/her sole, and
for the joint mechanism (300), the rotation range is determined so that a range of a plantar-flexion motion and a dorsiflexion motion of an ankle joint of the wearer is regulated.

## Patentansprüche

1. Stützapparat (10), mit:
einem ersten Element (101), das konfiguriert ist, an einem Teil eines Körpers befestigt zu werden, der entlang eines ersten Knochenteils eines Trägers gelegen ist;
einem zweiten Element (200), das konfiguriert ist, an einem Teil eines Körpers befestigt zu werden, der entlang eines zweiten Knochenteils des Trägers gelegen ist; und
einem Gelenkmechanismus (300), der konfiguriert ist, das erste und das zweite Element (101, 200) abzustützen, sodass das erste und das zweite Element (101, 200) in Bezug aufeinander um eine Drehachse drehen können, wobei
der Stützapparat (10) ferner ein Passungs-Einsetzelement (410, 420) aufweist, das konfiguriert ist, um in den Gelenkmechanismus (300) auf eine solche Weise passungs-eingesetzt zu werden, dass das Passungs-Einsetzelement (410, 420) an dem Gelenkmechanismus (300) fixiert und davon entfernt werden kann,
der Gelenkmechanismus (300)
einen Passungs-Einsetzaufnahmeteil (310c, 310e), der einstückig mit dem ersten Element (101) vorgesehen ist, wobei der Passungs-Einsetzaufnahmeteil (310c, 310e) konfiguriert ist, dem Passungs-Einsetzelement (410, 420) zu ermöglichen, in den Gelenkmechanismus (300) passungs-eingesetzt zu werden; und
einen Einstellungsteil (320) aufweist, der einstückig mit dem zweiten Element (200) vorgesehen ist, wobei der Einstellungsteil (320) um die Drehachse in dem zweiten Element (200) angeordnet ist, und
der Einstellungsteil (320), welcher auf eine einstückige Weise mit einer Drehung des zweiten Elements (200) um die Drehachse dreht, auf einer Trajektorie der Drehung des Einstellungsteils (320) mit einem Vorsprungsteil (411, 421) des Passungs-Einsetzelements (410, 420), das an dem Passungs-Einsetzaufnahmeteil (310c, 310e) fixiert ist, in Oberflächenkontakt gelangt, sodass ein Bereich der Drehung des zweiten Elements (200) eingestellt ist, wobei sich der Vorsprungsteil (411, 421) in Bezug auf die Drehachse in einer Umfangsrichtung erstreckt, **dadurch gekennzeichnet, dass**
das Passungs-Einsetzelement (410, 420), welches in den Passungs-Einsetzaufnahmeteil (310c, 310e) passungs-eingesetzt ist, gemäß einem gewünschten Einstelldrehbereich aus einem Satz einer Vielzahl von Passungs-Einsetzelementen (410, 420) ausgewählt ist, wobei die Vielzahl von Passungs-Einsetzelementen (410, 420) Vorsprungsteile (411, 421) aufweisen, die Vorsprungslängen haben, die voneinander verschieden sind, und
der Gelenkmechanismus einen Schwenkarm (350, 360) aufweist, der konfiguriert ist, das Passungs-Einsetzelement (410, 420) in einer Richtung zu dem Passungs-Einsetzaufnahmeteil (310c, 310e) herunterzudrücken, nachdem das Passungs-Einsetzelement (410, 420) in den Passungs-Einsetzaufnahmeteil (310c, 310e) eingesetzt ist.

2. Stützapparat (10) nach Anspruch 1, wobei das Passungs-Einsetzelement (410, 420) in der Lage ist, in einem Zustand, in welchem das erste und das zweite Element (101, 200) bereits an dem Träger befestigt sind, in den Passungs-Einsetzaufnahmeteil (310c, 310e) passungs-eingesetzt zu werden.

3. Stützapparat (10) nach Anspruch 1 oder 2, wobei das Passungs-Einsetzelement (410, 420)
ein erstes Passungs-Einsetzelement (410), das einen ersten Vorsprungsteil (411) aufweist, der konfiguriert ist, mit dem Einstellungsteil (320) an einem Ende einer Drehbewegung davon in Kontakt zu gelangen; und
ein zweites Passungs-Einsetzelement (420) aufweist, das einen zweiten Vorsprungsteil (421) aufweist, der konfiguriert ist, mit dem Einstellungsteil (320) an dem anderen Ende der Drehbewegung davon in Kontakt zu gelangen.

4. Stützapparat (10) nach Anspruch 1, wobei der Gelenkmechanismus (360, 424) einen Positionierungsmechanismus (366) zum Stoppen des Schwenkarms (360) in einer vorbestimmten Position aufweist.

5. Stützapparat (10) nach einem der Ansprüche 1 bis 4, wobei
eines des ersten und des zweiten Elements (101, 200) ein Unterschenkelrahmen (101) ist, der an einem Unterschenkel des Trägers befestigt ist,
das andere des ersten und des zweiten Elements (101, 200) eine Sohlenplatte (200) ist, auf welcher der Träger/die Trägerin seine/ihre Sohle platziert, und
der Drehbereich für den Gelenkmechanismus (300) bestimmt ist, sodass ein Bereich einer Plantarflexionsbewegung und einer Dorsiflexionsbewegung eines Sprunggelenks des Trägers eingestellt ist.

## Revendications

1. Attelle (10) comprenant :
un premier élément (101) configuré pour être fixé à une partie d'un corps positionné le long d'une première partie d'os d'un utilisateur ;
un second élément (200) configuré pour être fixé à une partie d'un corps positionné le long d'une seconde partie d'os de l'utilisateur ; et
un mécanisme de joint (300) configuré pour supporter les premier et second éléments (101, 200) de sorte que les premier et second éléments (101, 200) peuvent tourner l'un par rapport à l'autre autour d'un axe de rotation, dans laquelle :
l'attelle (10) comprend en outre un élément de montage par insertion (410, 420) configuré pour être monté par insertion dans le mécanisme de joint (300) de sorte que l'élément de montage par insertion (410, 420) peut être fixé sur et retiré du mécanisme de joint (300),
le mécanisme de joint (300) comprend :
une partie de réception de montage par insertion (310c, 310e) prévue de manière solidaire avec le premier élément (101), la partie de réception de montage par insertion (310c, 310e) étant configurée pour permettre à l'élément de montage par insertion (410, 420) d'être monté par insertion dans le mécanisme de joint (300) ; et
une partie de régulation (320) prévue de manière solidaire avec le second élément (200), la partie de régulation (320) étant disposée autour de l'axe de rotation dans le second élément (200), et
la partie de régulation (320) qui tourne autour de l'axe de rotation d'une manière intégrée avec une rotation du second élément (200), établit le contact de surface avec une partie en saillie (411, 421) de l'élément de montage par insertion (410, 420) fixée sur la partie de réception de montage par insertion (310c, 310e) sur une trajectoire de la rotation de la partie de régulation (320), de sorte qu'une plage de la rotation du second élément (200) est régulée, la partie en saillie (411, 421) s'étendant dans une direction circonférentielle par rapport à l'axe de rotation, **caractérisée en ce que** :
l'élément de montage par insertion (410, 420), qui est monté par insertion dans la partie de réception de montage par insertion (310c, 310e), est sélectionné parmi un ensemble d'une pluralité d'éléments de montage par insertion (410, 420) selon une plage de rotation régulée, la pluralité d'éléments de montage par insertion (410, 420) comprenant des parties en saillie (411, 421) ayant des longueurs en saillie différentes les unes des autres, et
le mécanisme de joint comprend un levier (350, 360) configuré pour comprimer l'élément de montage par insertion (410, 420) vers le bas dans une direction vers la partie de réception de montage par insertion (310c, 310e) après que l'élément de montage par insertion (410, 420) a été inséré dans la partie de réception de montage par insertion (310c, 310e).

2. Attelle (10) selon la revendication 1, dans laquelle l'élément de montage par insertion (410, 420) peut être monté par insertion dans la partie de réception de montage par insertion (310c, 310e) dans un état dans lequel les premier et second éléments (101, 200) sont déjà fixés à l'utilisateur.

3. Attelle (10) selon les revendications 1 ou 2, dans lequel l'élément de montage par insertion (410, 420) comprend :
un premier élément de montage par insertion (410) comprenant une première partie en saillie (411) configurée pour venir en contact avec la partie de régulation (320) au niveau d'une extrémité de son mouvement rotatif ; et
un second élément de montage par insertion (420) comprenant une seconde partie en saillie (421) configurée pour venir en contact avec la partie de régulation (320) au niveau de l'autre extrémité de son mouvement rotatif.

4. Attelle (10) selon la revendication 1, dans lequel le mécanisme de joint (360, 424) comprend un mécanisme de positionnement (366) pour arrêter le levier (360) dans une position prédéterminée.

5. Attelle (10) selon l'une quelconque des revendications 1 à 4, dans laquelle :
l'un des premier et second éléments (101, 200) est un bâti de jambe (101) fixé à une jambe de l'utilisateur,
l'autre des premier et second éléments (101, 200) est une plaque de semelle (200) sur laquelle l'utilisateur place sa semelle, et
pour le mécanisme de joint (300), la plage de rotation est déterminée de sorte qu'une plage d'un mouvement de flexion plantaire et d'un mouvement de flexion dorsale d'une articulation de cheville de l'utilisateur est régulée.
